# EUROPÄISCHE PATENTSCHRIFT

(11) **EP 2 731 637 B1**
(45) Veröffentlichungstag und Bekanntmachung des Hinweises auf die Patenterteilung: **11.11.2020**
(21) Anmeldenummer: 12762522.6
(22) Anmeldetag: 12.07.2012
(51) Int. Cl.: A61P 11/00, A61L 31/08, A61L 31/04

(54) **ZUSAMMENSETZUNG ZUR ERZEUGUNG EINER VORÜBERGEHENDEN DARMOKKLUSION**
COMPOSITION FOR PRODUCING A TEMPORARY INTESTINAL OBSTRUCTION
COMPOSITION POUR LA RÉALISATION D'UNE OCCLUSION INTESTINALE TEMPORAIRE

(30) Priorität: 13.07.2011 AT 10262011
(43) Veröffentlichungstag der Anmeldung: 21.05.2014
(73) Patentinhaber: Bischof, Georg, 1180 Wien (AT)
(72) Erfinder: Bischof, Georg, 1180 Wien (AT)
(74) Vertreter: Ellmeyer, Wolfgang
(86) Internationale Anmeldenummer: PCT/AT2012/050102
(87) Internationale Veröffentlichungsnummer: WO 2013/006886

(56) Entgegenhaltungen:
- WO-A1-96/22789
- WO-A1-97/22372
- WO-A1-2011/085424
- WO-A2-2008/103891
- WO-A2-2009/137446
- US-A1- 2006 222 596
- US-A1- 2008 215 036

## Beschreibung

Die vorliegende Erfindung betrifft das Gebiet der medizinischen Endoskopie und Endochirurgie, genauer gesagt jenes der Enteroskopie und -chirurgie.

Endoskopie ist ein seit vielen Jahrzehnten etabliertes Diagnoseverfahren in der Human- und Veterinärmedizin. Die hierfür verwendeten Endoskope haben sich kontinuierlich weiterentwickelt und ermöglichen heutzutage nicht nur einfaches Beleuchten und Abbilden des Körperinneren, z.B. unter Verwendung von Lichtleiterfasern, sondern sind auch für die Durchführung minimalinvasiver operativer Eingriffe ausgerüstet. So umfassen moderne Endoskope neben der Faseroptik beispielsweise Luftinsufflatoren oder Gaspumpen, Irrigatoren, Absaugpumpen sowie flexible Werkzeuge, wie z. B. Kanülen zur Injektion, Greif- oder Schneidwerkzeuge oder Drahtelektroden zur Koagulation mit elektrischem Strom. Zur Einführung der für den jeweiligen Eingriff erforderlichen Gerätschaften weist das Endoskop mehrere Kanäle auf.

Speziell bei enteroskopischen Eingriffen im Darm von Mensch und Tier treten, trotz vorhergehender Gabe von Abführmitteln, häufig Komplikationen aufgrund von die zu untersuchende und/oder operativ zu behandelnde Stelle passierendem Stuhl auf, was den Vorgang nicht nur erschwert und verzögert, sondern im Falle von operativen Eingriffen, z.B. zur Gewebeentnahme oder -entfernung, auch ein Infektionsrisiko für den Patienten darstellt. Somit wäre es wünschenswert, den Darm während eines endoskopischen Eingriffs temporär verschließen zu können.

In der Literatur finden sich einige Lösungen für diese Aufgabe. Der Hauptanspruch der WO 2008/103891 A2 ist zwar allgemein auf die Ausbildung eines Polymerpfropfens "an einer Stelle in einem Säugetier" abgestellt, indem eine viskose Polymerzusammensetzung bei Körpertemperatur verfestigen gelassen wird, deren einziger, im Rest der Anmeldung beschriebener Anwendungszweck ist jedoch der Verschluss von Arterien, d.h. Hämostase. Grundlage der dortigen Offenbarung sind revers thermosensitive Polymere, d.h. Polymere, die bei Raumtemperatur wasserlöslich sind, bei Körpertemperatur jedoch aus der Lösung ausfallen. Beispiele sind Poloxamere, z.B. solche, die von BASF unter dem Handelsnamen Pluronics® vertrieben werden.

Weitere Beispiele finden sich etwa in WO 2009/137446 A2 und US 2008/215036 A1, wo ebenfalls Poloxamere beschrieben werden. In der WO 2009/044403 A2 werden Copolymere von Hydroxyfettsäuren enthaltende Zusammensetzungen, die bei Kontakt mit physiologischen Flüssigkeiten ihre Viskosität erhöhen, zur Gewebereparatur beschrieben. Und die WO 96/22789 A1 offenbart aus Alginat, Chitosan und Poly-L-aminosäuren ausgewählte biokompatible Polymere zum Verschluss zum Gefäßen.

Ein Nachteil dieser Ausführungsformen ist jedoch, dass die jeweilige Zusammensetzung nur schwer an der Stelle einer gewünschten Okklusion im Darm befördert werden kann, ohne zumindest teilweise wieder von dort abzufließen, bevor sich der Pfropfen gebildet hat. Wenn sich der Pfropfen aufgrund von Quellung ausbilden soll, wie z.B. im Fall der WO 96/22789 A1, ist zudem eine relativ große Flüssigkeitsmenge in den Darm zu pumpen, wodurch ein Abfließen von der gewünschten Einsatzstelle noch wahrscheinlicher wird.

Dazu kommt, dass Mechanismen, die auf Blutgefäße abgestimmt sind, wo die jeweilige Zusammensetzung mit verhältnismäßig großen Mengen an Flüssigkeit, also Blut, in Kontakt kommt, um rasche Temperatur- oder Viskositätsänderungen herbeizuführen, im Darm so nicht funktionieren: Patienten werden vor Eingriffen im Darm üblicherweise Abführmittel verabreicht, so dass die Zusammensetzung nur mit äußerst geringen, an der Darmwand anhaftenden Flüssigkeitsmengen in Kontakt kommt.

Ziel der Erfindung war daher die Bereitstellung einer Zusammensetzung, mit der die obigen Nachteile ausgemerzt werden können.

### OFFENBARUNG DER ERFINDUNG

Dieses Ziel erreicht die Erfindung durch Bereitstellung einer Zusammensetzung zur Erzeugung einer vorübergehenden Okklusion des Darms eines Säugetiers, wobei die Zusammensetzung fließfähig und an einer gewünschten Stelle im Darm zu einem festen Pfropfen verfestigbar ist, dessen Struktur zur anschließenden, zumindest teilweisen Aufhebung der Okklusion veränderbar ist, wobei die Zusammensetzung eine fließfähige Suspension in einem Lösungsmittel oder Lösungsmittelgemisch ist oder umfasst,
dadurch gekennzeichnet, dass die Zusammensetzung Folgendes umfasst:
a) eine Suspension eines Feststoffs in Wasser oder einem wässrigen Lösungsmittelgemisch mit einem Gehalt an Wasser, der um eine Menge X oberhalb der Fließgrenze der Suspension liegt;
b) ein wasserentziehendes Mittel in einer Menge, die ausreicht, um die Menge X an Wasser zu binden, so dass durch den Wasserentzug die Fließgrenze der Suspension überschritten wird; und
c) ein Mittel zum Passivieren des wasserentziehenden Mittels.

Mittels eines solchen Zusammensetzung lässt sich an der gewünschten Stelle im Darm ein fester Pfropfen aus dem sedimentierten Feststoff erzeugen, indem die Passivierung des wasserentziehenden Mittels aufgehoben wird. Letzteres bindet daraufhin zumindest die Menge X an Wasser, die die Fließgrenze der Suspension des Feststoffs übersteigt, wodurch dieser sedimentiert, sich verfestigt und dabei den Darm verschließt. Das Prinzip der Pfropfenbildung beruht dabei nicht oder nicht vorwiegend auf einer Quellung oder Gelierung des Feststoffs selbst, sondern auf einem völlig anderen Mechanismus, nämlich Sedimentation. Je nach Art des wasserentziehenden Mittels kann zwar mitunter der Wasserentzug durch Quellung herbeigeführt werden, wie nachstehend ausgeführt wird, dies muss aber nicht der Fall sein. Somit kann die Flüssigkeitsmenge der Zusammensetzung als Ganzes deutlich geringer sein, als dies nach dem Stand der Technik zumeist möglich ist.

Unter Suspension ist hierin ein stabiles, nicht spontan sedimentierendes Gemisch aus einem Feststoff und Wasser zu verstehen, die je nach Teilchengröße des suspendierten Feststoffs Suspendierhilfen enthalten kann. Die Teilchengröße des Feststoffs in der Suspension ist nicht speziell eingeschränkt und kann bis hinunter zu kolloidalen Dimensionen reichen, in welchem Fall zumeist keine Suspendierhilfe erforderlich ist.

Das Lösungsmittel der Suspension kann neben Wasser auch ein oder mehrere physiologisch unbedenkliche organische Lösungsmittel enthalten, d.h. ein wässriges Lösungsmittelgemisch sein. Als Beispiele für die in Frage kommenden organischen Lösungsmittel seien Alkohole und Glykole, z.B. Glycerin oder Polyethylenglykol, Ether, z.B. Glykolether, und Ester, z.B. Glykolester, erwähnt.

Die Konzentration des Feststoffs in der wässrigen Suspension wird in bevorzugten Ausführungsformen so gewählt, dass sie nicht mehr als 5 %, noch bevorzugter nicht mehr als 3 %, noch bevorzugter nicht mehr als 1 % unterhalb der Fließgrenze liegt, so dass erstens nur eine sehr geringe Menge des wasserentziehenden Mittels und zweitens nur eine sehr kurze Zeitspanne notwendig ist, um die Zusammensetzung zu einem Pfropfen zu verfestigen. Dadurch tritt die Verfestigung sehr rasch ein, bevor die Zusammensetzung von Abgabestelle abfließen kann. Die die Fließgrenze der Suspension übersteigende Wassermenge X sind dabei vorzugsweise nur wenige Gramm oder auch nur 1 Gramm oder weniger. Der Betrag der Menge X hängt in manchen Ausführungsformen auch davon ab, wie weit die geplante Einsatzstelle im Darm vom Darmausgang entfernt liegt, wie dies später noch näher ausgeführt wird.

Der suspendierte Feststoff ist nicht speziell eingeschränkt, solange er in der Lage ist, mit Wasser oder einem wässrigen Lösungsmittelgemisch eine Suspension zu bilden, die sich bei einer definierten Konzentration durch Sedimentation verfestigt. Dafür kommen beispielsweise diverse natürliche und künstliche Polymere, wie z.B. Polysaccharide, granuläre Kunststoffe und Gemische davon, aber auch anorganische Feststoffe, z. B. mineralische Feststoffe, vor allem weiche, nicht abrasive, feinkörnige Pulver, wie z.B. Kreide und Tonmineralien, in Frage. Unter "Feststoff" ist hierin auch ein Gemisch zweier oder mehrerer Feststoffe mit entsprechender Wirkung zu verstehen.

Vorzugsweise ist oder umfasst der suspendierte Feststoff jedoch ein Polysaccharid, noch bevorzugter eines auf Stärke- oder Cellulosebasis, da diese kostengünstig und für den Körper unschädlich sind und Suspensionen mit gut steuerbaren Eigenschaften bilden. Insbesondere wird nicht-kaltlösliche Stärke, z.B. granuläre, native Kartoffelstärke, Tapiokastärke, Maisstärke, Weizenstärke oder Reisstärke als Feststoff verwendet, da diese feinteilige, sphärische, nichtabrasive Naturstoffe mit scharf definierten Fließpunkten sind und neben den obigen Vorteilen kostengünstig erhältlich und physiologisch völlig unbedenklich ist.

Das wasserentziehende Mittel ist nicht speziell eingeschränkt, und dessen Funktion kann auf verschiedenen physikalischen oder chemischen Mechanismen beruhen. So kann das Wasser beispielsweise durch chemische Reaktionen gebunden werden, z.B. durch spontane Hydrolyse von labilen Bindungen, wie z.B. Orthoestern oder Acetalen, wobei die Suspension und/oder das wasserentziehende Mittel selbst zu diesem Zweck weitere Reaktionspartner wie etwa Katalysatoren, z.B. eine schwache Säure oder Base, enthalten kann. Diese sollten wiederum, genau wie die dabei gebildeten Reaktionsprodukte und generell sämtliche Komponenten der erfindungsgemäßen Zusammensetzung, unschädlich und physiologisch unbedenklich sein. Als weitere Reaktionen zur Bindung des Wassers kommen beispielsweise Hydratation und Quellung von Feststoffen, vorzugsweise hygroskopischen Feststoffen, in Frage, wofür neben den später näher erläuterten organischen Polymeren z.B. auch Natriumsulfat und andere wasserfreie anorganische Salze in Frage kommen.

Das wasserentziehende Mittel b) kann mitunter auch in Form einer festen Tablette oder dergleichen vorliegen, die z.B. mit dem Greifwerkzeug des Endoskops zur Einsatzstelle transportiert wird, wonach über einen Endoskopkanal die Suspension a) auf die Tablette aufgespritzt wird, wodurch der suspendierte Feststoff sedimentiert und gegebenenfalls gleichzeitig das wasserentziehende Mittel quillt, um den Pfropfen zu bilden (siehe unten).

Theoretisch kommt für die Zwecke hierin auch eine Kombination aus einer oben definierten Feststoffsuspension und einem wasserentziehenden Mittel, das seine Wirkung auf mechanische Weise entfaltet, in Frage, wie z.B. ein wasserentziehendes Schwämmchen oder eine Keramik- oder Glasfritte, die wiederum getrennt voneinander an die gewünschte Stelle im Darm befördert und erst dort in Kontakt gebracht werden. Die Passivierung besteht dabei in eben dieser getrennten Zufuhr zum Einsatzort. Aufgrund der höheren Aufwands sind solche Lösungen der technischen Aufgabe allerdings nicht bevorzugt.

Vorzugsweise ist oder umfasst das wasserentziehende Mittel ein in Wasser quellbares Polymer, das beispielsweise aus jenen ausgewählt sein kann, die als Zerfallshilfsmittel oder Tablettensprengmittel zum Einsatz kommen, z.B. Stärke oder Cellulose oder Derivate davon, Alginate, Dextrane, vernetztes Polyvinylpyrrolidon etc. Weitere Beispiele sind so genannte Superabsorber, d.h. stark wasserabsorbierende Polymere, wie sie etwa auch in diversen Hygieneartikeln zum Einsatz kommen, beispielsweise auf Acrylnitril, Acrylsäure, Acrylamid, Polyvinylalkohol basierende Polymere, wie z.B. Polyacrylat/Polyacrylamid-, Ethylen/Maleinsäureanhydrid- oder Acrylsäure/Natriumacrylat-Copolymere, vernetztes Polyethylenoxid usw. Noch bevorzugter ist das wasserentziehende Mittel ein Polysaccharid, noch bevorzugter Stärke, insbesondere Natriumcarboxymethylstärke, da diese wiederum unschädlich und vor allem kostengünstig sind.

Bei der Quellung erfährt ein solches wasserentziehendes Mittel auf Stärkebasis außerdem eine starke Volumszunahme und vergrößert dabei das Volumen des hauptsächlich aus dem sedimentierten Feststoff gebildeten Pfropfens - selbst wenn das Mengenverhältnis zwischen Feststoffsuspension und wasserentziehendem Mittel sehr groß ist -, wodurch der Pfropfen das Darmlumen leicht ausfüllen und so verschließen kann. Dadurch kann wiederum die Flüssigkeitsmenge der Zusammensetzung als Ganzes gering gehalten werden.

Die Menge an wasserentziehendem Mittel in der erfindungsgemäßen Zusammensetzung hängt direkt mit der Konzentration an suspendiertem Polymer und der Fließgrenze der Suspension zusammen. Sie muss jedenfalls ausreichen, um den Wasserüberschuss X über der Konzentration bei der Fließgrenze binden zu können. Vorzugsweise reicht sie aus, um zumindest 1 %, noch bevorzugter zwischen 1 % und 10 %, noch bevorzugter zwischen 1 % und 5 %, noch bevorzugter zwischen 1 % und 3 %, des Wassers in der Zusammensetzung zu binden.

Dabei ist auch die bei der Bindung des Wassers üblicherweise frei werdende Wärmemenge zu berücksichtigen, die keine übermäßige Erwärmung der sich verfestigenden Zusammensetzung bewirken sollte. Diese Wärmemenge kann jedoch auch gezielt genutzt werden, wie nachstehend ausführlicher beschrieben wird.

In manchen bevorzugten Ausführungsformen der Erfindung stellt zumindest ein Teil des wasserentziehenden Mittels gleichzeitig zumindest einen Teil des suspendierten Feststoffs dar, wie dies nachstehend noch näher ausgeführt wird.

Die Art des Mittels zur Passivierung des wasserentziehenden Mittels ist nicht speziell eingeschränkt, solange wirksam verhindert wird, dass das wasserentziehende Mittel mit dem Wasser der Suspension reagieren kann. Beispielsweise kann die Passivierung unmittelbar vor Verwendung oder auch erst im Darm deaktivierbar sein. So kann das Passivierungsmittel etwa eine chemische Modifizierung des wasserentziehenden Mittels, z.B. eine Schutzgruppe, sein, die erst entfernt werden muss, bevor es aktiv werden und mit Wasser reagieren kann. Beispielsweise kann ein quellbares Polysaccharid mit hydrophoben Gruppen modifiziert sein, die vor ihrer Abspaltung den Zutritt von Wasser zum Polysaccharid verhindern.

Beruht die wasserentziehende Wirkung selbst ebenfalls auf einer chemischen Reaktion mit Wasser, sollte die Deaktivierung des Passivierungsmittels zumindest gleich rasch wie die Reaktion des aktivierten wasserentziehenden Mittels mit Wasser ablaufen. Die Aktivierung kann mitunter durch Reaktion eines zusätzlichen Reaktanten mit dem passivierten wasserentziehenden Mittel erfolgen, der in der Suspension enthalten ist, wie z. B. einer Säure oder Base.

In bevorzugten Ausführungsformen ist das Passivierungsmittel jedoch keine chemische Modifikation, sondern eine Beschichtung um das wasserentziehende Mittel, die zur Aktivierung entfernt werden muss. Beispielsweise kann eine wasserlösliche und/ oder hydrolysierbare Beschichtung verwendet werden, die sich bei Kontakt mit der wässrigen Suspension allmählich auflöst und so das wasserentziehende Mittel freisetzt. In solchen Fällen ist die Zusammensetzung vorzugsweise ein Zwei-Komponenten-System, das erst unmittelbar vor der Anwendung zusammengemischt werden darf. Die im Falle von exothermer Solvatation frei werdende Lösungswärme beschleunigt die Auflösung der Beschichtung ebenso zusätzlich wie die beim Transport durch den Endoskopkanal erfolgende Erwärmung der Zusammensetzung. Darüber hinaus binden die in Lösung gehenden Moleküle der Beschichtung bereits einen Teil des Wassers, so dass sich die Suspension des Polymers der Fließgrenze annähert, bevor das wasserentziehende Mittel aktiv wird. Die Beschichtung als Passivierungsmittel dient in solchen Fällen selbst ebenfalls als wasserentziehendes Mittel gemäß vorliegender Erfindung.

In besonders bevorzugten Ausführungsformen besteht die Beschichtung aus einem Material, das bei den im Darm herrschenden Temperaturen schmilzt, wobei wiederum die Erwärmung im Endoskop zu berücksichtigen ist. Speziell wenn die Stelle der geplanten Okklusion weit vom Darmausgang entfernt liegt, ist die für die Einführung des Endoskops benötigte Zeitspanne sehr lang, z.B. bis zu einer halben Stunde. Aufgrund der im Darm normalerweise herrschenden Temperatur von rund 37 °C kann sich das Endoskop in dieser Zeit mitunter auf eine Temperatur von über 30 °C, z.B. auf etwa 35 °C erwärmen. Daher wird vorzugsweise ein Material der Beschichtung verwendet, das nicht unter 35 °C, noch bevorzugter nicht unter 35,5 °C, noch bevorzugter nicht unter 36 °C, noch bevorzugter nicht unter 36,5 °C und in Sonderfällen erst bei etwa 37 °C, schmilzt.

Das Material der Beschichtung ist vorzugsweise aus natürlichen oder synthetischen Wachsen und Fetten ausgewählt, wobei natürliche aufgrund ihrer guten Verträglichkeiten und bekannten Wirkung gemäß vorliegender Erfindung besonders bevorzugt wird. Besonders bevorzugt werden Materialien, wie sie zur Beschichtung von Suppositorien oder Zäpfchen eingesetzt werden, wie etwa Hartfett, das einen Schmelzbereich nahe der menschlichen Körpertemperatur aufweist. Beispiele dafür sind aus Palmkern- und Kokosfett gewonnenes Hartfett, das vorwiegen aus Laurinsäure besteht, deren Schmelzpunkt durch gezielte Steuerung des Grads einer anschließenden Veresterung mit Glycerin, d.h. der OH-Zahl des so erhaltenen Esters, präzise steuerbar ist. Ein weiteres bevorgtes Beispiel ist Kakaobutter, die Schmelzbereiche zwischen 30 und 38 °C aufweisen kann, noch bevorzugte Kakaobutter in der β-Modifikation. Aufgrund der guten Steuerbarkeit des Schmelzpunkts wird Hartfett besonders bevorzugt.

Generell kann jedoch der Schmelzpunkt der jeweiligen Beschichtung von einem einschlägigen Fachmann auch ohne übermäßiges Experimentieren durch Herstellung von Mischungen der oben genannten Substanzen mit geeigneten höher schmelzenden Wachsen, Fetten oder Harzen feinjustiert werden.

Einem vollständigen Abschmelzen der Beschichtung und somit Freilegung des wasserentziehenden Mittels während des Transports durch den erwärmten Endoskopkanal kann jedoch auch durch geeignete Wahl der Beschichtungsdicke entgegengewirkt werden. Bei geeigneten Druckverhältnissen sollte die Zusammensetzung binnen weniger Sekunden das Endoskop passiert haben, so dass eine entsprechend dicke Beschichtung noch nicht zur Gänze abgeschmolzen ist. Dasselbe Prinzip gilt selbstverständlich auch für eine wasserlösliche Beschichtung.

Die Dicke der Beschichtung sollte daher ausreichen, um einerseits einen sicheren Transport durch den Endoskopkanal zu gewährleisten, anderseits jedoch eine anschließende rasche Entfernung im Darm zu ermöglichen.

Je nach Art der Passivierung kann das wasserentziehende Mittel in der Suspension des Feststoffs ebenfalls suspendiert sein, oder es kann unmittelbar vor der Verwendung der erfindungsgemäßen Zusammensetzung darin aufgewirbelt und anschließend sofort durch den Endoskopkanal gepumpt werden. Vorzugsweise bilden die Feststoffsuspension und das wasserentziehende Mittel eine zumindest für einige Minuten stabile Suspension, um eine homogene Verteilung zu gewährleisten, weswegen sehr feinteilige wasserentziehende Mittel ebenso bevorzugt werden wie eine entsprechend gut suspendierbare Beschichtung derselben, sofern eine solche zur Pasivierung vorgesehen ist.

In besonders bevorzugten Ausführungsformen stellt, wie bereits erwähnt, zumindest ein Teil des suspendierten Feststoffs gleichzeitig zumindest einen Teil des wasserentziehenden Mittels dar. Das heißt, der suspendierte Feststoff oder ein Teil davon ist selbst in der Lage, Wasser zu binden, sobald die Passivierung aufgehoben wurde. Das Mittel zur Passivierung des wasserentziehenden Mittels kann dabei wiederum eine Beschichtung oder chemische Modifikation des Letzteren, wie oben beschrieben, umfassen. Vorzugsweise besteht das Mittel zur Passivierung in diesen Ausführungsformen der Erfindung allerdings darin, das Wasser und das wasserentziehende Mittel vor der Verwendung voneinander räumlich getrennt bereitzustellen und erst im Darm oder unmittelbar vor der Beförderung der erfindungsgemäßen Zusammensetzung in den Darm miteinander zu vermischen.

Die Zusammensetzung liegt in solchen Ausführungsformen vor Verwendung vorzugsweise als Zwei-Komponenten-System vor, das als erste Komponente eine Suspension des wasserentziehenden Mittels in einem, vorzugsweise im Wesentlichen wasserfreien, aber mit Wasser ausreichend gut mischbaren, physiologisch unbedenklichen Lösungsmittel und als zweite Komponente Wasser oder ein wasserhaltiges Gemisch umfasst. Die beiden Komponenten werden unmittelbar vor oder während der Verwendung miteinander vermischt, wodurch sich eine Suspension des als wasserentziehendes Mittel dienenden Feststoffs in einem somit auch Wasser enthaltenden Lösungsmittelgemisch bildet, deren Wassergehalt anfangs um den Betrag X über der Fließgrenze liegt, durch die Reaktion des wasserentziehenden Mittels sich jedoch mehr oder weniger rasch der Fließgrenze annähert, bis diese nach Bindung der Wassermenge X schließlich erreicht ist, so dass wiederum Sedimentation des Feststoffs erfolgt.

Besonders bei Vermischung der beiden Komponenten des Zwei-Komponenten-Systems außerhalb des Darms, was erfindungsgemäß bevorzugt wird, da auf diese Weise nur ein Kanal des Endoskops für die Zufuhr der erfindungsgemäßen Zusammensetzung zur Einsatzstelle benötigt wird, sind die Mengenverhältnisse in Abhängigkeit von der Entfernung der Einsatzstelle vom Darmausgang sehr genau zu wählen. Der Transport der vermischten Komponenten zur Einsatzstelle muss rascher erfolgen, als das wasserentziehende Mittel zur Bindung der Wassermenge X benötigt, damit es nicht bereits im Endoskopkanal zur Sedimentation kommt. Ein konkretes Beispiel für eine solche Ausführungsform wird in den späteren Ausführungsbeispielen beschrieben.

Alternativ dazu kann die Vermischung der zwei Komponenten, wie erwähnt, auch erst an der Einsatzstelle erfolgen, was entweder zwei freie Endoskopkanäle oder eine Thixotropierung der Suspension erfordert, damit diese nicht von der Einsatzstelle abläuft, bevor sie mit Wasser in Kontakt kommt. Oder es kann ein Teil des als wasserentziehendes Mittel dienenden Feststoffs zusätzlich zur anfänglichen räumlichen Trennung auch durch eine Beschichtung oder chemische Modifikation passiviert sein, die erst im Darm, z.B. durch Schmelzen, deaktiviert wird.

Neben den oben definierten Komponenten kann eine erfindungsgemäße Zusammensetzung natürlich beliebige sonstige Bestandteile umfassen, solange dadurch die Wirkungsweise der Erfindung nicht beeinträchtigt wird. Beispiele sind etwa Suspendierhilfen, Viskositätsregler, oberflächenaktive Stoffe oder Haftvermittler zur Erhöhung der Anhaftung des Pfropfens an der Darmwand, wie z. B. Naturharz-Derivate, Casein und andere tierische Proteine. Weiters können in manchen Ausführungsformen der erfindungsgemäßen Zusammensetzung auch Schäumer und Schaumstabilisatoren enthalten sein, da der Pfropfen bisweilen auch von einem festen Schaum gebildet werden kann. Zu diesem Zweck können beispielsweise Körnchen, die aus dem wasserentziehenden Mittel und einem oder mehreren Schäumern bestehen, von einer Wachs- oder ähnlichen Beschichtung umgeben sein, so dass diese beim Schmelzen oder bei Auflösung der Beschichtung gleichzeitig aktiviert werden.

In einem zweiten Aspekt betrifft die Erfindung ein Verfahren zur Erzeugung einer vorübergehenden Okklusion des Darms eines Säugetiers unter Verwendung der Zusammensetzung gemäß dem ersten Aspekt der Erfindung, das Folgendes umfasst:
i) das Transportieren der Zusammensetzung an eine gewünschte Stelle im Darm;
ii) das Aufheben der Passivierung des wasserentziehenden Mittels, um dadurch Sedimentation auszulösen und die Zusammensetzung an dieser Stelle zu einem festen Pfropfen zu verfestigen, der den Darm verschließt; und gegebenenfalls
iii) das Verändern der Struktur des Pfropfens, um die Okklusion zumindest teilweise wieder aufzuheben;
   wobei Schritt iii) entfallen kann, wenn sich die Struktur des Pfropfens im Darm im Lauf der Zeit von selbst, d.h. ohne äußeres Zutun, verändert und so die Okklusion zumindest teilweise wieder aufgehoben wird.

In bevorzugten Ausführungsformen des erfindungsgemäßen Verfahrens wird zur zumindest teilweisen Aufhebung der Darmokklusion in Schritt iii) dem Pfropfen einfach zusätzliches Wasser zugeführt, um diesen wieder zu verflüssigen, indem die Konzentration wiederum unter die Fließgrenze gesenkt wird, so dass die Bestandteile des Pfropfens wieder von der Einsatzstelle abfließen können.

Eine mögliche, allgemeine Herangehensweise zur Herstellung einer Zusammensetzung gemäß dem ersten Aspekt der Erfindung und insbesondere zur Einstellung der Mengenverhältnisse wird anhand eines konkreten, relativ einfachen Beispiels erläutert. So gilt es für den einschlägigen Fachmann zunächst, Komponente a) auszuwählen, wobei er sich beispielsweise für Maisstärke (wie in den späteren Ausführungsbeispielen 1 und 2) entscheiden würde. Mittels einfacher Vorversuche bestimmt man nun die Fließgrenze einer Suspension der zur Verfügung stehenden Stärkepulver-Charge, z.B. durch portionsweises Zudosieren des Stärkepulvers zu einem gerührten Wasserkörper bekannten Gewichts, z.B. Portionen von 0,5 g Stärke zu 100 g Wasser. Für Maisstärke wird dabei wohl ungefähr ein Wert von 42 Gew.-% für die Fließgrenze der wässrigen Suspension erhalten werden.

Sodann wird ein wasserentziehendes Mittel ausgewählt, z.B. Natriumcarboxymethylstärke, und bestimmt, wie viel Wasser das Mittel pro Gewichtseinheit zu binden imstande ist, z.B. indem 1 g des wasserentziehenden Mittels zu 10 ml Wasser zugesetzt, kurz gerührt und danach wieder abfiltriert wird und anschließend die Restwassermenge ausgewogen wird. Dabei wird beispielsweise ein Wasseraufnahmevermögen von rund 5 g pro 1 g Carboxymethylstärke festgestellt werden.

Danach könnte der Fachmann beispielsweise festlegen, dass er als Feststoffsuspension der erfindungsgemäßen Zusammensetzung eine 40%ige Suspension von Maisstärke in Wasser (d.h. 40 g Stärke in 60 g Wasser) einsetzen möchte. Um die Konzentration der Stärke von 40 % auf 42 % zu erhöhen und so durch Überschreiten der Fließgrenze Sedimentation der Stärke auszulösen, müssen vom wasserentziehenden Mittel rund 5 g Wasser gebunden werden, zumal 40 g Stärke in 55 g Wasser 42,1 Gew.-% entsprechen.

Folglich ist mindestens jene Menge an wasserentziehendem Mittel zuzusetzen, die imstande ist, 5 g Wasser zu binden, was gemäß der obigen Bestimmung etwa 1 g Carboxymethylstärke entspricht.

Dementsprechend stünde die Menge X in diesem Beispielfall für 5 g Wasser, und als Mindestmenge an Komponente b) wäre rund 1 g Carboxymethylstärke erforderlich.

Nachstehend wird die vorliegende Erfindung nun anhand von konkreten Ausführungsbeispielen beschrieben.

### BEISPIELE

### Beispiel 1

In eine 40%ige wässrige Suspension (bezogen auf die Trockensubstanz) von nativer Maisstärke wurden 3 Gew.-% der Stärketrockensubstanz einer granulären Natriumcarboxymethylstärke zugesetzt, wie sie im Handel z.B. als Primojel® von DMV Fronterra oder Ultramyl® von Gustav Parmentier erhältlich ist, die zuvor mit einer Beschichtung aus Kakaobutter passiviert worden war. Beim Erwärmen der hergestellten Suspension auf 37 °C verfestigte sich die Suspension ab etwa 35 °C zu einem festen Sediment, das seine Fließfähigkeit völlig einbüßte. Bei Zugabe von Wasser gewann das feste Sediment, sobald es eine Konzentration von unter 35 % erreichte, seine Fließfähigkeit rasch wieder zurück.

### Beispiel 2

In eine 40%ige wässrige Suspension (bezogen auf die Trockensubstanz) von nativer Maisstärke wurden 3 Gew.-% der Stärketrockensubstanz einer granulären Natriumcarboxymethylstärke zugesetzt, die zuvor mit einer Beschichtung aus Hartfett passiviert worden war. Zusätzlich wurden 1 Gew.-% der Stärketrockensubstanz eines Suspensionsstabilisators, nämlich kaltlösliche Maisquellsärke, in die Suspension eingerührt. Die Suspension neigte bei Lagerung unterhalb von 30 °C nicht zur Ausbildung eines Sediments. Beim Erwärmen der hergestellten Suspension auf 37 °C verfestigte sich die Suspension ab etwa 36 °C zu einem festen Sediment, das seine Fließfähigkeit völlig einbüßte. Bei Zugabe von Wasser gewann das feste Sediment, sobald es eine Konzentration von unter 35 % erreichte, seine Fließfähigkeit rasch wieder zurück.

### Beispiel 3

In eine 38%ige wässrige Suspension (bezogen auf die Trockensubstanz) von nativer Kartoffelstärke wurden 3 Gew.-% der Stärketrockensubstanz einer granulären Natriumcarboxymethylstärke zugesetzt, die zuvor mit einer Beschichtung aus Kakaobutter passiviert worden war. Beim Erwärmen der hergestellten Suspension auf 37 °C verfestigte sich die Suspension ab etwa 35 °C zu einem festen Sediment, das seine Fließfähigkeit völlig einbüßte. Bei Zugabe von Wasser gewann das feste Sediment, sobald es eine Konzentration von unter 35 % erreichte, seine Fließfähigkeit rasch wieder zurück.

### Beispiel 4

Eine 38%ige wässrige Suspension (bezogen auf die Trockensubstanz) von nativer Kartoffelstärke wurde 30 min lang bei 50 °C gerührt und anschließend auf 25 °C abgekühlt. Daraufhin wurden 3 Gew.-% der Stärketrockensubstanz einer granulären Natriumcarboxymethylstärke zugesetzt, die zuvor mit einer Beschichtung aus Kakaobutter passiviert worden war. Die Suspension neigte bei Lagerung unterhalb von 30 °C nicht zur Ausbildung eines Sediments. Beim Erwärmen der hergestellten Suspension auf 37 °C verfestigte sich die Suspension ab etwa 35 °C zu einem festen Sediment, das seine Fließfähigkeit völlig einbüßte. Bei Zugabe von Wasser gewann das feste Sediment, sobald es eine Konzentration von unter 33 % erreichte, seine Fließfähigkeit rasch wieder zurück.

### Beispiel 5

Die Suspension aus Beispiel 1 wurde über eine Kanüle in ein künstliches Darmsegment aus Schweinedarm eingeleitet, das bei 37 °C gelagert wurde. Es bildete sich rasch ein festes Sediment aus, welches das Darmsegment vollständig verschloss. Nach einem Zeitraum von 30 Minuten konnte das feste Sediment mittels Spülung mit Wasser leicht wieder aus dem Darmsegment entfernt werden.

### Beispiel 6

100 ml einer Suspension von granulärer Natriumcarboxymethylstärke, wie sie im Handel beispielsweise als Primojel® von DMV Fronterra oder Ultramyl® von Gustav Parmentier erhältlich ist, in Glycerin mit einer Viskosität von rund 1500 mPa.s wird als Komponente 1 eines Zwei-Komponenten-Systems über eine Schlauchleitung zu einem Ypsilonstück gepumpt. Die Natriumcarboxymethylstärke dient dabei sowohl als suspendierter Feststoff als auch als wasserentziehendes Mittel. Als Komponente 2 des Systems werden 20 ml Wasser über eine weitere Schlauchleitung ebenfalls zu diesem Ypsilonstück gepumpt. Das Mittel zur Passivierung des wasserentziehenden Mittels besteht demnach einerseits im Vorsehen einer räumlichen Trennung der beiden Komponenten der erfindungsgemäßen Zusammensetzung, d.h. von wasserentziehendem Mittel und Wasser, und andererseits in der Suspendierung des ersteren in Glycerin, das sich erst mit dem Wasser vermischen muss, damit die erfindungsgemäße Zusammensetzung ihre Wirkung entfalten kann.

Die Komponenten 1 und 2 vermischen sich im Ypsilonstück, wobei die Viskosität des Gemischs erheblich niedriger liegt als jene der Glycerin-Susoension, nämlich bei etwa 200 mPa.s. Dieses niedrigviskose Gemisch wird über eine Sonde durch den Arbeitskanal eines Endoskops innerhalb von wenigen Sekunden an die gewünschte Stelle im Darm transportiert. Innerhalb von etwa 10 Sekunden nach dem Vermischen nimmt die Viskosität des Gemischs aufgrund von Wasseraufnahme der Körner sehr rasch zu, wodurch sich das Gemisch zu einem festen Sediment aus durch die Wasseraufnahme stark vergrößerten Körnern der Carboxymethylstärke verfestigt. Dieses Sediment bildet einen Pfropfen, der das Segment des Darms vollständig verschließt. Durch einfache Zugabe von ausreichend Wasser wird das feste Sediment nach Abschluss der Untersuchung wieder verflüssigt und so die Okklusion aufgehoben.

Somit stellt die vorliegende Erfindung Zusammensetzungen bereit, mittels derer der Darm eines Patienten zur Durchführung eines medizinischen Eingriffs auf einfache, kostengünstige und physiologisch völlig unbedenkliche Weise okkludiert werden kann.

## Patentansprüche

1. Zusammensetzung zur Verwendung zur Erzeugung einer vorübergehenden Okklusion des Darms eines Säugetiers, wobei die Zusammensetzung fließfähig und an einer gewünschten Stelle im Darm zu einem festen Pfropfen verfestigbar ist, dessen Struktur zur anschließenden, zumindest teilweisen Aufhebung der Okklusion veränderbar ist, wobei die Zusammensetzung eine fließfähige Suspension in einem Lösungsmittel oder Lösungsmittelgemisch ist oder umfasst,
**dadurch gekennzeichnet, dass** die Zusammensetzung Folgendes umfasst:
a) eine Suspension eines Feststoffs in Wasser oder einem wässrigen Lösungsmittelgemisch mit einem Gehalt an Wasser, der um eine Menge X oberhalb der Fließgrenze der Suspension liegt;
b) ein wasserentziehendes Mittel in einer Menge, die ausreicht, um die Menge X an Wasser zu binden, so dass durch den Wasserentzug die Fließgrenze der Suspension überschritten wird;
wobei vor der Verwendung
c) das wasserentziehende Mittel durch chemische Modifikation oder eine Beschichtung passiviert ist.

2. Zwei-Komponenten-System zur Herstellung einer Zusammensetzung durch Vermischen der beiden Komponenten, die vor der Verwendung räumlich voneinander getrennt vorliegen, wobei die dadurch herstellbare Zusammensetzung
i) zur Verwendung zur Erzeugung einer vorübergehenden Okklusion des Darms eines Säugetiers dient,
ii) fließfähig und an einer gewünschten Stelle im Darm zu einem festen Pfropfen verfestigbar ist, dessen Struktur zur anschließenden, zumindest teilweisen Aufhebung der Okklusion veränderbar ist, und
iii) die Zusammensetzung eine fließfähige Suspension in einem wässrigen Lösungsmittelgemisch ist,
wobei das Zwei-Komponenten-System Folgendes umfasst:
a) als erste Komponente eine Suspension eines Feststoffs in einem im Wesentlichen wasserfreien, aber mit Wasser mischbaren, physiologisch unbedenklichen Lösungsmittel oder Lösungsmittelgemisch, wobei der Feststoff als wasserentziehendes Mittel dient und in dem Lösungsmittel oder Lösungsmittelgemisch einer Menge enthalten ist, die ausreicht, um eine Menge X an Wasser zu binden; und
b) als zweite Komponente Wasser in einer solchen Menge, dass beim Vermischen mit der ersten Komponente die Wassermenge die Fließgrenze der Suspension des Feststoffs in dem dabei gebildeten wässrigen Lösungsmittelgemisch um die Menge X übersteigt.

3. Zusammensetzung nach Anspruch 1 oder Zwei-Komponenten-System nach Anspruch 2, **dadurch gekennzeichnet, dass** der suspendierte Feststoff eine granuläre, native Stärke ist oder umfasst.

4. Zusammensetzung nach einem der Ansprüche 1 bis 3 oder oder Zwei-Komponenten-System nach Anspruch 2 oder 3, **dadurch gekennzeichnet, dass** das wasserentziehende Mittel bzw. der als wasserentziehendes Mittel dienende Feststoff ein in Wasser quellbares Polysaccharid ist oder umfasst.

5. Zusammensetzung oder Zwei-Komponenten-System nach Anspruch 4, **dadurch gekennzeichnet, dass** das in Wasser quellbare Polysaccharid Natriumcarboxymethylstärke ist.

6. Zusammensetzung nach einem der Ansprüche 1 und 3 bis 5, **dadurch gekennzeichnet, dass** zumindest ein Teil des suspendierten Feststoffs gleichzeitig zumindest einen Teil des wasserentziehenden Mittels darstellt.

7. Zusammensetzung nach einem der Ansprüche 1 und 3 bis 6 oder Zwei-Komponenten-System nach einem der Ansprüche 2 bis 5, **dadurch gekennzeichnet, dass** das wasserentziehende Mittel bzw. der als wasserentziehendes Mittel dienende Feststoff vor der Verwendung durch eine Beschichtung passiviert ist.

8. Zusammensetzung oder Zwei-Komponenten-System nach Anspruch 7, **dadurch gekennzeichnet, dass** die Beschichtung aus einem Material besteht, das bei den im Darm herrschenden Temperaturen schmilzt.

9. Zusammensetzung oder Zwei-Komponenten-System nach Anspruch 8, **dadurch gekennzeichnet, dass** das Material der Beschichtung nicht unter 35 °C schmilzt.

10. Zusammensetzung oder Zwei-Komponenten-System nach Anspruch 9, **dadurch gekennzeichnet, dass** das Material der Beschichtung nicht unter 36 °C schmilzt.

11. Zusammensetzung oder Zwei-Komponenten-System nach einem der Ansprüche 7 bis 10, **dadurch gekennzeichnet, dass** das Material der Beschichtung aus Hartfett und Kakaobutter ausgewählt ist.

12. Zwei-Komponenten-System nach einem der Ansprüche 2 bis 5 und 7 bis 11, **dadurch gekennzeichnet, dass** das mit Wasser mischbare, physiologisch unbedenkliche Lösungsmittel Glycerin ist oder umfasst.

## Claims

1. A composition for forming a temporary intestinal occlusion in a mammal, said composition being flowable and solidifiable to form a solid plug at a predetermined site in the intestine, the structure of said plug being changeable to allow for a subsequent, at least partial removal of said occlusion, said composition being or comprising a flowable suspension in a solvent or mixed solvent,
**characterized in that** said composition comprises the following:
a) a suspension of a solid in water or an aqueous mixed solvent, the suspension having a water content which exceeds the flow limit of the suspension by an amount X;
b) a dehydrating agent in an amount which is sufficient to bind the amount X of water, so that, as a consequence of dehydration, the flow limit of the suspension is exceeded;and
c) said dehydrating agent is passivated by means of chemical modification or a coating.

2. A two-component system for producing a composition by mixing the two components which, before use, remain spatially separated from one another, the thus obtainable composition being
i) suitable for forming a temporary intestinal occlusion in a mammal,
ii) flowable and solidifiable to form a solid plug at a predetermined site in the intestine, the structure of said plug being changeable to allow for a subsequent, at least partial removal of said occlusion, and
iii) a flowable suspension in an aqueous mixed solvent,
said two-component system comprising:
a) as a first component, a suspension of a solid in an essentially anhydrous, but water-miscible, physiologically acceptable solvent or mixed solvent, said solid acting as a dehydrating agent and being contained in said solvent or mixed solvent in an amount which is sufficient to bind an amount X of water, and
b) as a second component, water in such an amount that, when mixed with the first component, the amount of water exceeds the flow limit of the suspension of the solid in the thus formed aqueous mixed solvent by the amount X.

3. The composition according to claim 1 or the two-component system according to claim 2, **characterized in that** the suspended solid is or comprises granular native starch.

4. The composition according to any one of the claims 1 to 3 or the two-component system according to claim 2 or claim 3, **characterized in that** the dehydrating agent or the solid acting as a dehydrating agent is or comprises a water-swellable polysaccharide.

5. The composition or the two-component system according to claim 4, **characterized in that** said water-swellable polysaccharide is sodium carboxymethyl starch.

6. The composition according to any one of the claims 1 and 3 to 5, **characterized in that** at least a portion of the suspended solid at the same time constitutes at least a portion of the dehydrating agent.

7. The composition according to any one of the claims 1 and 3 to 6, or the two-component system according to any one of the claims 2 to 5 **characterized in that**, before use, the dehydrating agent or the solid acting as dehydrating agent is passivated by means of a coating.

8. The composition or the two-component system according to claim 7, **characterized in that** the coating consists of a material which melts at the temperatures prevailing in the intestine.

9. The composition or the two-component system according to claim 8, **characterized in that** the coating material does not melt at temperatures below 35 °C.

10. The composition or the two-component system according to claim 9, **characterized in that** the coating material does not melt at temperatures below 36 °C.

11. The composition or the two-component system according to any one of the claims 7 to 10, **characterized in that** the coating material is selected from hard fat and cocoa butter.

12. The two-component system according to any one of the claims 2 to 5 and 7 to 11, **characterized in that** the water-miscible, physiologically acceptable solvent is or comprises glycerol.

## Revendications

1. Composition pour former une occlusion intestinale temporaire chez un mammifère, ladite composition pouvant s'écouler et pouvant être solidifiée pour former un bouchon solide à un endroit prédéterminé dans l'intestin, la structure dudit bouchon pouvant être modifiée pour permettre une élimination subséquente, au moins partielle, de ladite occlusion, ladite composition étant ou comprenant une suspension pouvant s'écouler dans un solvant ou un mélange de solvants,
**caractérisée en ce que** ladite composition comprend:
a) une suspension d'un solide dans l'eau ou dans un mélange de solvants aqueux, la suspension ayant une teneur en eau qui dépasse la limite d'écoulement de la suspension d'une quantité X;
b) un agent déshydratant en une quantité suffisante pour lier la quantité X d'eau, de sorte que, par suite de la déshydratation, la limite d'écoulement de la suspension est dépassée; et
c) ledit agent déshydratant est passivé au moyen d'une modification chimique ou d'un revêtement.

2. Système à deux composants pour la production d'une composition en mélangeant ces deux composants qui, avant d'être utilisés, restent séparés spatialement l'un de l'autre, la composition ainsi obtenue
i) étant utile pour former une occlusion intestinale temporaire chez un mammifère,
ii) pouvant s'écouler et pouvant être solidifiée pour former un bouchon solide à un endroit prédéterminé de l'intestin, la structure dudit bouchon pouvant être modifiée pour permettre une élimination subséquente, au moins partielle, de ladite occlusion, et
iii) étant une suspension pouvant s'écouler dans un mélange de solvants aqueux,
ledit système à deux composantes comprenant:
a) comme un premier composant, une suspension d'un solide dans un solvant ou un mélange de solvants essentiellement anhydre, mais miscible à l'eau, et physiologiquement acceptable, ledit solide servant d'un agent déshydratant et étant contenu dans ledit solvant ou mélange de solvants en une quantité qui est suffisante pour lier la quantité X d'eau, et
b) comme un deuxième composant, de l'eau en quantité telle que, lorsqu'elle est mélangée au premier composant, la quantité d'eau dépasse d'une quantité X la limite d'écoulement de la suspension du solide dans le mélange de solvants aqueux ainsi formé.

3. Composition selon la revendication 1 ou système à deux composants selon la revendication 2, caractérisé/e en ce que le solide en suspension est ou comprend de l'amidon natif granulaire.

4. Composition selon l'une quelconque des revendications 1 à 3 ou système à deux composants selon la revendication 2 ou 3, caractérisé/e en ce que l'agent déshydratant ou le solide agissant servant d'agent déshydratant est ou comprend un polysaccharide gonflable dans l'eau.

5. Composition ou système à deux composants selon la revendication 4, caractérisé/e en ce que ledit polysaccharide gonflable à l'eau est le carboxyméthylamidon sodique.

6. Composition selon l'une quelconque des revendications 1 et 3 à 5, **caractérisée en ce qu'**au moins une partie du solide en suspension constitue en même temps au moins une partie de l'agent déshydratant.

7. Composition selon l'une quelconque des revendications 1 et 3 à 6 ou système à deux composants selon l'une quelconque des revendications 2 à 5, caractérisé/e en ce que, avant son utilisation, l'agent déshydratant ou le solide servant d'agent déshydratant est passivé au moyen d'un revêtement.

8. Composition ou système à deux composants selon la revendication 7, caractérisé/e en ce que le revêtement est constitué d'un matériau qui fond aux températures dans l'intestin.

9. Composition ou système à deux composants selon la revendication 8, caractérisé/e en ce que le matériau de revêtement ne fond pas à des températures inférieures à 35 °C.

10. Composition ou système à deux composants selon la revendication 9, caractérisé/e en ce que le matériau de revêtement ne fond pas à des températures inférieures à 36 °C.

11. Composition ou système à deux composants selon l'une quelconque des revendications 7 à 10, caractérisé/e en ce que le matériau de revêtement est choisie parmi la graisse dure et le beurre de cacao.

12. Composition ou système selon l'une des revendications 2 à 5 et 7 à 11, caractérisé/e en ce que le solvant physiologiquement acceptable et miscible à l'eau est ou comprend du glycérol.
